# EUROPEAN PATENT APPLICATION

(11) **EP 0 551 019 A1**
(43) Date of publication of application: **14.07.1993**
(21) Application number: 92311891.3
(22) Date of filing: 31.12.1992
(51) Int. Cl.: C07D 311/10

(54) **Catalytic process of producing coumarin and derivatives thereof**

(30) Priority: 07.01.1992 JP 494/92
(71) Applicant: SUMITOMO CHEMICAL COMPANY, LIMITED, Osaka-shi, Osaka (JP); N.E. CHEMCAT CORPORATION, Minato-ku, Tokyo 105 (JP)
(72) Inventor: Nishida, Yoshitaka, Niihama-shi, Ehime (JP); Sakai, Kiyomi, Niihama-shi, Ehime (JP); Shirafuji, Tamio, Niihama-shi, Ehime (JP); Yokoyama, Masakazu, Niihama-shi, Ehime (JP); Muroi, Takashiro, Ushiku-shi, Ibaraki (JP)
(74) Representative: Dixon, Donald Cossar

(57) **Abstract**

A 3-(2-oxocyclohexyl)propionic acid ester of a specified formula, which has a terminal Cl-4 alkyl, is cyclized and dehydrogenated to form the corresponding coumarin or derivative thereof in the presence as catalyst of 0.1-5 wt%, by wt of the ester, of palladium supported mostly (preferably at least 90% of the Pd) in the surface portion of a carrier, which can be an element of Group IIA, IIIA or IVA or a compound thereof, e.g. carbon, used as a powder of particle size 1-50 µm.

The reaction is at 100-350°C (preferably 230-280°C), and can be in a solvent, and optionally with a co-catalyst.

The products are obtained in better yield than with prior forms of the catalyst, and are useful in the perfume industry.

## Description

This invention relates to a process of producing coumarin or derivatives thereof from 3-(2-oxocyclohexyl)-propionic acid esters. Coumarin and its derivatives, together with 3,4-dihydrocoumarin and its derivatives, are an important compound in the perfume industry and are also useful as an intermediate for dyes, agricultural chemicals or drugs.

Known processes for obtaining coumarin or derivatives thereof include a process comprising charging a hydrogenation-dehydrogenation catalyst, e.g., palladium, and a 3-(2-oxocyclohexyl)propionic acid esters before the reaction and then heating the mixture to conduct cyclization and dehydrogenation, as disclosed in U.S. Patent 3,442,910; and a process comprising the cyclization and dehydrogenation of a 3-(2-oxocyclohexyl)propionic acid esters in the presence of a noble metal catalyst, e.g., palladium, in combination with a co-catalyst, such as barium sulfate or nickel oxide, as disclosed in JP-A-60-181082 (the term "JP-A" as used herein means an "unexamined published Japanese patent application"). The noble metal catalyst used in these processes, e.g., palladium, is a catalyst system comprising palladium or a similar metal uniformly supported on a carrier.

However, these conventional processes utilizing cyclization and dehydrogenation of a 3-(2-oxocyclohexyl)-propionic acid esters in the presence of a conventional palladium-on-carrier catalyst do not always attain a high yield of coumarin or its derivatives.

An object of the present invention is to provide a process for producing coumarin or derivatives thereof from a 3-(2-oxocyclohexyl)propionic acid esters in an increased yield.

As a result of extensive investigation, we have now found that the yield of coumarin can be increased by carrying out the dehydrogenation by using a palladium-on-carrier catalyst in which most of the palladium is in the surface portion of the carrier, called an "egg shell catalyst", instead of the conventional catalyst in which the palladium is uniformly distributed in the carrier up to the central portion thereof. The present invention has been completed based on this finding.

The present invention relates to a process for producing coumarin or derivatives thereof represented by the formula (2): 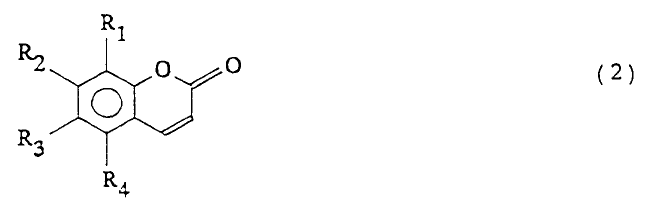
wherein R₁, R₂, R₃ and R₄ each represents a hydrogen atom, a methyl group, or an ethyl group, provided that at least two of them represent hydrogen atoms,
the process comprising cyclizing and dehydrogenating a 3-(2-oxocyclohexyl)propionic acid ester represented by formula (1): 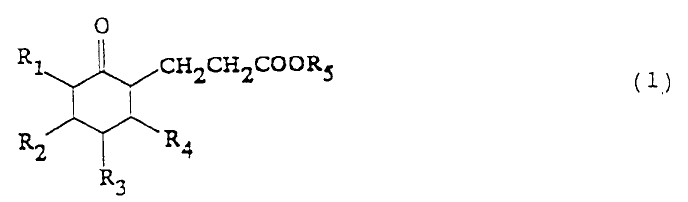
wherein R₁, R₂, R₃ and R₄ are as defined above; and R₅ represents an alkyl group having from 1 to 4 carbon atoms,
in the presence of a palladium-on-carrier catalyst, the palladium-on-carrier catalyst being an egg shell palladium-on-carrier catalyst.

Examples of the 3-(2-oxocyclohexyl)propionic acid esters which can be used in the present invention include methyl 3-(2-oxoycyclohexyl)propionate, butyl 3-(2-oxocyclohexyl)propionate, methyl 3-(3-methyl-2-oxocyclohexyl)propionate, methyl 3-(5-methyl-2-oxocyclohexyl)propionate, propyl 3-(4-ethyl-2-oxocyclohexyl)propionate, propyl 3-(3,4-dimethyl-2-oxocyclohexyl)propionate, propyl 3-(3,4-dimethyl-2-oxocyclohexyl)propionate, methyl 3-(3,5-dimethyl-2-oxocyclohexyl)propionate, and methyl 3-(3-ethyl-6-methyl-2-oxocyclohexyl)propionate, but the 3-(2-oxocyclohexyl)propionic acid esters are not limited to these examples. Among these, methyl 3-(2-oxocyclohexyl)propionate is preferably used in the present invention.

The catalyst which can be used in the present invention is an egg shell palladium-on-carrier catalyst. In a preferred embodiment of the present invention, the catalyst is a solid metallic catalyst comprising at least one carrier selected from an element of Group IIA, IIIA or IVA and a compound thereof, e.g., carbon, alumina, silica gel or barium sulfate, having supported thereon from about 0.5 to 10% by weight of palladium based on the amount of the carrier. In the egg shell catalyst used in the present invention, at least 90% of the palladium is preferably present in a surface portion of the carrier within a depth of up to 0.5 µm from the surface thereof. The distribution of palladium in the carrier can be analyzed by Electron Probe Microanalyzer (EPMA) or Electron Microscope. The particle size of the catalyst is generally from 1 to 50 µm, and preferably from 10 to 20 µm.

The palladium-on-carrier catalyst is generally used in an amount of about from 0.1 to 5% by weight (about from 0.005 to 0.25% by weight as palladium metal), and preferably of about from 0.3 to 2% by weight (about from 0.015 to 0.1% by weight as palladium metal), based on the amount of the starting 3-(2-oxocyclohexyl)propionic acid esters. If the amount of the catalyst is too small, the reaction activity tends to become too low. Too large an amount tends to have excessive reaction activity, resulting in an increase of by-products.

The cyclization and dehydrogenation of a 3-(2-oxocyclohexyl)propionic acid ester is generally carried out at a temperature ranging from about 100 to 350°C, and preferably about 230 to 280°C. If the temperature is too low, the reaction activity tends to become low. At temperatures exceeding about 350°C, the starting compound tends to decompose.

The reaction may be performed in a solvent. Examples of suitable solvents include phenyl ether, benzyl ether, methyl α-naphthyl ether, ethylnaphthalene, dimethylbiphenyl, dodecane, tetradecane, tetralin, acetophenone, phenyl propyl ketone, methyl benzoate, and dimethyl glutamate. The amount of the solvent is generally from 0.5 to 10 times by weight the weight of the 3-(2-oxocyclohexyl)propionic acid esters.

In carrying out the reaction, the starting compound and the catalyst, along with a co-catalyst (e.g., magnesium trisilicate, zirconia, metallic chromium) and a solvent, if desired, are charged into a reactor before the reaction, and the mixture is heated at a prescribed temperature for several hours to several tens of hours. The amount of any co-catalyst is generally about from 0.001 to 3% by weight, preferably about from 0.5 to 2.5% by weight, based on the amount of the 3-(2-oxocyclohexyl)propionic acid esters.

The reaction time is generally from about 5 to 50 hours, and preferably about from 10 to 40 hours.

As a result of the reaction of methyl 3-(2-oxocyclohexyl)propionate, a 3,4-dihydrocoumarin is usually obtained in a yield of from about 30 to 40% and a coumarin is usually obtained in a yield of about 30 to 45%, together with by-products, such as o-ethylphenol, methyl dihydrocinnamate and octahydrocoumarin

By the process of the present invention, coumarin or derivatives thereof can be obtained in a higher yield than in conventional processes.

The present invention is now illustrated in greater detail with reference to an Example, but it should be understood that the present invention is not construed as being limited thereto. The percentages in the Example and Comparative Example are by weight unless otherwise indicated.

### EXAMPLE 1

In a four-necked flask were charged 300 g of methyl 3-(2-oxcyclohexyl)propionate and 2.1 g of 5% palladium-on-activated carbon egg shell catalyst in which 5% of palladium based on the carrier amount was supported on the carrier ("Water-containing 5% Pd-on-carbon Powder E-Type" produced by N.E. Chemcat Co.). The mixture was heated in a nitrogen atmosphere at 250°C for 10 hours with stirring at 240 rpm. The temperature was elevated up to 270°C over a period of 1 hour, and the reaction was continued at that temperature for 15 hours with stirring at 500 rpm. After completion of the reaction, the catalyst was removed by filtration. Gas chromatography analysis of the filtrate revealed that the conversion of methyl 3-(2-oxocyclohexyl)propionate was 100% and the yields of coumarin and 3,4-dihydrocoumarin were 38.4% by mol and 35.2% by mol, respectively, based on the amount of the starting methyl 3(2-oxocyclohexyl)propionate.

### COMPARATIVE EXAMPLE 1

In a four-necked flask were charged 300 g of methyl 3-(2-oxocyclohexyl)propionate and 2.1 g of conventional 5% palladium-on-activated carbon in which 5% of palladium based on the carrier amount was uniformly present throughout the carrier ("Water-containing 5% Pd-on-carbon Powder N-Type" produced by N.E. Chemcat Co.). The mixture was heated in a nitrogen atmosphere at 250°C for 10 hours with stirring at 240 rpm. The temperature was elevated up to 270°C over a period of 1 hour, and the reaction was continued at that temperature for 15 hours with stirring at 500 rpm. After completion of the reaction, the catalyst was removed by filtration. Gas chromatography analysis of the filtrate revealed that the conversion of methyl 3-(2-oxocyclohexyl)-propionate was 99.9% and the yields of coumarin and 3,4-dihydrocoumarin were 32.2% by mol and 36.8% by mol, respectively, based on the amount of the starting methyl 3-(2-oxocyclohexyl)propionate.

## Claims

1. A process of producing coumarin or derivatives thereof represented by formula (2): 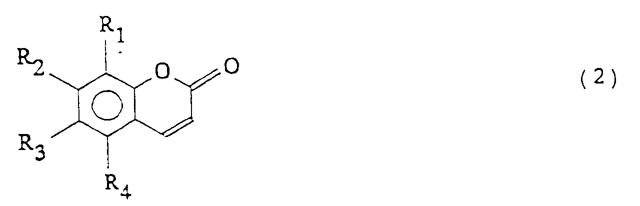 wherein R₁, R₂, R₃ and R₄ each represents a hydrogen atom, a methyl group or an ethyl group, provided that at least two of them represent hydrogen atoms,
said process comprising cyclizing and dehydrogenating 3-(2-oxocyclohexyl)propionic acid esters represented by formula (1): 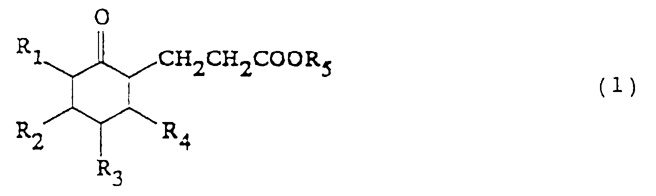 wherein R₁, R₂, R₃ and R₄ are as defined above; and R₅ represents an alkyl group having from 1 to 4 carbon atoms,
in the presence of a palladium-on-carrier catalyst, characterised in that said catalyst is an "egg shell" palladium-on-carrier catalyst in which most of the palladium is in the surface portion of the carrier.

2. A process as claimed in Claim 1, wherein said egg shell catalyst is a catalyst in which at least 90% of the palladium is present in a surface portion of the carrier within a depth of up to 0.5 µm from the surface thereof.

3. A process as claimed in Claim 1 or 2, wherein said egg shell catalyst contains from 0.5 to 10% by weight of palladium based on the amount of the carrier.

4. A process as claimed in Claim 1, 2 or 3, wherein said egg shell catalyst is used in an amount of from 0.1 to 5% by weight based on the amount of said 3-(2-oxocyclohexyl)propionic acid esters.

5. A process as claimed in any of Claims 1 to 4, wherein said cyclizing and dehydrogenating reaction is carried out at a temperature of from 230 to 280°C.

6. A process as claimed in any of Claims 1 to 5, wherein said 3-(2-oxocyclohexyl)propionic acid ester is methyl 3-(2-oxocyclohexyl)propionate.

7. A process as claimed in any preceding claim, which is carried out in a solvent.

8. A process as claimed in any preceding claim, wherein the carrier is an element of Group IIA, IIIA or IVA and/or a compound thereof.

9. A process as claimed in Claim 8, wherein said carrier is carbon, alumina, silica gel or barium sulfate.
